# EUROPEAN PATENT SPECIFICATION

(11) **EP 1 248 597 B1**
(45) Date of publication and mention of the grant of the patent: **30.03.2005**
(21) Application number: 00985666.7
(22) Date of filing: 21.12.2000
(51) Int. Cl.: A61K 9/12

(54) **PHARMACEUTICAL AEROSOL FORMULATION OF SALMETEROL AND FLUTICASONE PROPIONATE**
PHARMAZEUTISCHE AEROSOL FORMULIERUNG ENTHALTEND SALMETEROL UND FLUTICASON
FORMULATION PHARMACEUTIQUE EN AEROSOL DE SALMETEROL ET DE PROPIONATE DE FLUTICASONE

(30) Priority: 24.12.1999 GB 9930878; 28.07.2000 GB 0018686
(43) Date of publication of application: 16.10.2002
(73) Proprietor: GLAXO GROUP LIMITED, Greenford, Middlesex UB6 ONN (GB)
(72) Inventor: CRIPPS, Alan Leslie, Glaxo Wellcome plc, Ware, Hertfordshire SG12 0DP (GB); JOHNSON, Paul, Glaxo Wellcome plc, Ware, Hertfordshire SG12 0DP (GB)
(74) Representative: Giddings, Peter John
(86) International application number: PCT/GB2000/004939
(87) International publication number: WO 2001/047493

(56) References cited:
- EP-A- 0 416 951
- WO-A-00/30607
- WO-A-94/13262
- WO-A-98/24420
- WO-A-98/56349
- WO-A-99/65464
- US-A- 5 653 962
- US-A- 5 919 827
- US-A- 6 004 537

## Description

### Background of the Invention

### Field of the Invention

The present invention relates to pharmaceutical formulations for use in the administration of medicaments by inhalation. In particular, this invention relates to pharmaceutical formulations of fluticasone propionate and salmeterol or a pharmaceutically acceptable salt thereof (such as the xinafoate) for use in pressurised metered dose inhalers (MDI's). The invention also relates to methods for their preparation and to their use in therapy.

### Description of the Background Art

Inhalers are well known devices for administering pharmaceutically active materials to the respiratory tract by inhalation. Such active materials commonly delivered by inhalation include bronchodilators such as β2 agonists and anticholinergics, corticosteroids, anti-allergies and other materials that may be efficiently administered by inhalation, thus increasing the therapeutic index and reducing side effects of the active material.

4-hydroxy-α¹-[[[6-(4-phenylbutoxy)hexyl]amino]methyl]-1,3-benzenedimethanol was described as one of a wide range of bronchodilators in GB-A-2140800. This compound is also known by the generic name of salmeterol, the xinafoate salt of which has become widely known as a highly effective treatment of inflammatory diseases, such as asthma and chronic obstructive pulmonary disease (COPD).

(6a, 11b, 16a, 17a)-6, 9-difluoro-11-hydroxy-16-methyl-3-oxo-17-(1-oxopropoxy) androsta-1, 4-diene-17-carbothioic acid, S-fluoromethyl ester was described as an antiinflammatory steroid by US Patent No. 4,335,121. This compound is also known by the generic name of fluticasone propionate and has since become widely known as a highly effective steroid in the treatment of inflammatory diseases, such as asthma and chronic obstructive pulmonary disease (COPD).

Coadministration of salmeterol and fluticasone propionate is believed to have significant advantages in the treatment and control of these inflammatory diseases.

Metered dose inhalers (MDI's) are the most common type of a wide range of inhaler types and utilise a liquefied propellant to expel droplets containing the pharmaceutical product to the respiratory tract as an aerosol. MDI formulations are generally characterised as solution formulations or suspension formulations.

The most commonly used aerosol propellants for medicaments have been Freon 11 (CCl₃F) in admixture with Freon 12 (CCl₂F₂) and Freon 114 (CF₂Cl.CF₂Cl). However, these propellants are now believed to provoke the degradation of stratospheric ozone and their use is now being phased out to eliminate the use of all CFC containing aerosol propellants. There is thus a need to provide an aerosol formulation for medicaments which employ so called 'ozone-friendly' propellants.

Hydrofluoroalkanes (HFAs; known also as hydrofluorocarbons or HFCs) contain no chlorine and are considered less destructive to ozone and these are proposed substitutes for CFCs. In particular, 1,1,1,2-tetrafluoroethane (HFA 134a) and 1,1,1,2,3,3,3-heptafluoropropane (HFA 227) have been acknowledged to be the best candidates for non-CFC propellants.

The efficiency of an aerosol device, such as an MDI, is a function of the dose deposited at the appropriate site in the lungs. Deposition is affected by several factors, of which one of the most important is the aerodynamic particle size. Solid particles and/or droplets in an aerosol formulation can be characterised by their mass median aerodynamic diameter (MMAD, the diameter around which the mass aerodynamic diameters are distributed equally). Particle deposition in the lung depends largely upon three physical mechanisms:
1. impaction, a function of particle inertia;
2. sedimentation due to gravity; and
3. diffusion resulting from Brownian motion of fine, submicrometer (<1µm) particles.

The mass of the particles determines which of the three main mechanisms predominates.

The effective aerodynamic diameter is a function of the size, shape and density of the particles and will affect the magnitude of forces acting on them. For example, while inertial and gravitational effects increase with increasing particle size and particle density, the displacements produced by diffusion decrease. In practice, diffusion plays little part in deposition from pharmaceutical aerosols. Impaction and sedimentation can be assessed from a measurement of the MMAD which determines the displacement. across streamlines under the influence of inertia and gravity, respectively.

Aerosol particles of equivalent MMAD and GSD (geometric standard deviation) have similar deposition in the lung irrespective of their composition. The GSD is a measure of the variability of the aerodynamic particle diameters.

For inhalation therapy there is a preference for aerosols in which the particles for inhalation have a diameter of about 0.5 to 5µm. Particles which are larger than 5µm in diameter are primarily deposited by inertial impaction in the oropharynx, particles 0.5 to 5µm in diameter, influenced mainly by gravity, are ideal for deposition in the conducting airways, and particles 0.5 to 3µm in diameter are desirable for aerosol delivery to the lung periphery. Particles smaller than 0.5µm may be exhaled.

Respirable particles are generally considered to be those with aerodynamic diameters less than 5µm. These particles, particularly those with a diameter of about 3µm, are efficiently deposited in the lower respiratory tract by sedimentation.

It has been recently demonstrated in patients with mild and severe airflow obstruction that the particle size of choice for a β2 agonist or anticholinergic aerosol should be approximately 3µm (Zaanen, P. et al, Int. J. Pharm. (1994) 107, 211-217, Int. J. Pharm. (1995) 114, 111-115, Thorax (1996), 51, 977-980.)

Many of the factors relevant to the MMAD of particles are relevant to droplets and the additional factors of rate of solvent evaporation, and surface tension are also important.

In suspension formulations, particle size in principle is controlled during manufacture by the size to which the solid medicament is reduced, usually by micronisation. However, if the suspended drug has the slightest solubility in propellant; a process known as Ostwald Ripening can lead to particle size growth. Also, particles may have tendency to aggregate, or adhere to parts of the MDI eg.canister or valve. The effect of Ostwald ripening and particularly of drug deposition may be particularly severe for potent drugs (including salmeterol xinafoate and fluticasone propionate) which need to be formulated in low doses. Solution formulations do not suffer from these disadvantages, but suffer from different ones in that particle or droplet size is both a function of rate of evaporation of the propellant from the formulation, and of the time between release of formulation from the canister and the moment of inhalation. Thus, it may be subject to considerable variability and is generally hard to control.

Besides its impact on the therapeutic profile of a drug, the size of aerosol particles has an important impact on the side effect profile of a drug. For example, it is well known that the oropharynx deposition of aerosol formulations of steroids can result in side effects such as candidiasis of mouth and throat. Furthermore, a higher systemic exposure to the aerosol particles due to deep lung penetration can enhance the undesired systemic effects of certain drugs. For example, the systemic exposure to certain steroids can produce side effects on bone metabolism and growth.

Suspension aerosol formulations containing salmeterol and fluticasone propionate are described in EP 0 416 951 A and US 5 653 962 A.

Formulations of R salmeterol are disclosed in US 5 919 827 A.

Solution formulations of medicaments are disclosed in WO 98/56349 A, WO 94/13262 A, WO 98/24420 A and WO 00/30607 A.

We have now invented a formulation of salmeterol or a pharmaceutically acceptable salt thereof and fluticasone propionate which eliminates or substantially mitigates some or all of the abovementioned disadvantages.

### Summary of the Invention

Thus, according to the present invention we provide a pharmaceutical aerosol formulation, comprising (i) salmeterol or a pharmaceutically acceptable salt thereof, (ii) fluticasone propionate, (iii) a hydrofluoroalkane (HFA) propellant, (iv) a low volatility component selected from glycerol and polyethylene glycol to increase the mass median aerodynamic diameter (MMAD) of the aerosol particles on actuation of the inhaler; and (v) ethanol as a solubilisation agent in sufficient quantity to solubilise the salmeterol or pharmaceutically acceptable salt thereof and fluticasone propionate in the formulation; and characterised in that the salmeterol or pharmaceutically acceptable salt thereof and fluticasone propionate are completely dissolved in the formulation.

### Detailed description of the Invention

The presence of the low volatility component in the solution formulation increases the fine particle mass (FPM) as defined by the content of stages 3-5 of an Andersen Cascade Impactor on actuation of the formulation relative to solutions formulations which omit this component. Solution formulations which omit the lower volatility component generally give rise to a particle size distribution which have a higher content of finer particles; such distributions generally do not match the distribution of suspension formulations and may therefore not be bio-equivalent.

Examples of HFA propelants include 1,1,1,2-tetrafluoroethane (HFA134a) and 1,1,1,2,3,3,3-heptafluoro-n-propane (HFA227) and mixtures thereof. The preferred propellant is 1,1,1,2-tetrafluoroethane (HFA134a). An alternative preferred propellant is 1,1,1,2,3,3,3-heptafluoro-n-propane (HFA227).

The low volatility component is glycerol or polyethyleneglycol (eg PEG200 or PEG400). Preferably the low volatility component is present in an amount of 0.5 to 3% (w/w)

The solubilisation agent is ethanol.

In a first embodiment of the invention we prefer salmeterol to be used in the form of the xinafoate salt. Salmeterol xinafoate may be prepared in two polymorphic forms known as Form I and Form II. Form I which has a melting endotherm at 140 °C may be prepared by precipitation from a hot methanolic solution of salmeterol xinafoate on addition to cold isopropanol as described in International Patent Application No. WO93/16031. Form II which has a melting endotherm at 125 °C may be prepared by supercritical fluid recrystallisation as described in International Patent Application No. WO95/01324. Preferably salmeterol xinafoate is employed as Form II polymorph since this form would be predicted to have a higher solubility. Alternatively salmeterol xinafoate may be employed as the Form I polymorph.

More particularly we prefer to use salmeterol xinafoate in the form of the purified enantiomer R-salmeterol xinafoate. Surprisingly we have found that R-salmeterol xinafoate in a polymorphic form obtainable by crystallisation from ether is significantly more soluble in mixtures of ethanol/HFA134a and ethanol/HFA227 than racemic salmeterol xinafoate. Without being limited by theory, this higher solubility may be attributed to the low crystal lattice energy as demonstrated by a melting endotherm at 95 °C (which is considerably lower than that of the two forms of salmeterol xinafoate mentioned above).

In a second embodiment of the invention we prefer to use salmeterol as the free base.

Surprisingly we have found that salmeterol base is substantially more soluble in mixtures of ethanol/HFA134a and ethanol/HFA227 than racemic salmeterol xinafoate or even R-salmeterol xinafoate. It is also of interest to use salmeterol base as R-salmeterol base.

Use of R-salmeterol xinafoate or base has the further advantage that it takes advantage of the higher potency of R-salmeterol relative to racemic salmeterol with the result that a lower concentration of the drug in solution is required.

In a third less preferred embodiment salmeterol is used as the sulphate salt. The preferred solubilising agent for salmeterol sulphate is propylene glycol.

As is apparent from the examples, formulations of salmeterol base and fluticasone propionate in ethanol and HFA134a or HFA227, particularly together with the a low volatility component such as glycerol or polyethyleneglycol, show particularly excellent delivery characteristics.

In the foregoing, except where otherwise indicated, drug quantities of salmeterol are given as appropriate for salmeterol base but it will be understood that for a salmeterol xinafoate or another pharmaceutically acceptable salt thereof an appropriate conversion to give a suitable weight of active principal in the delivered dose may be made. For example a dose of 25 µg of salmeterol equates to a dose of 36.3 µg of salmeterol xinafoate. It will also be understood that salmeterol may be used as the racemate (as is preferred) or in the form of an enantiomerically enriched (or purified) single R- or S-enantiomer. In the foregoing drug quantities are given as appropriate for racemic drug but it will be understood that adjustment of the dosage weight may be appropriate when a different ratio of enantiomers is employed. For example R-salmeterol may desirably be employed at one half of the normal dose of racemic salmeterol.

We prefer the formulation to be suitable for delivering a therapeutic amount of salmeterol and fluticasone propionate in one or two actuations. Preferably the formulation will be suitable for delivering 25-50 µg salmeterol per actuation, especially 25 µg per actuation of salmeterol (eg as xinafoate). Preferably, the formulation will be suitable for delivering 25-250µg fluticasone propionate per actuation, particularly 25µg, 50µg, 125µg or 250µg especially 25µg or 50µg per actuation of fluticasone propionate.

The formulation according to the invention will be used in association with a suitable metering valve. We prefer that the formulation is actuated by a metering valve capable of delivering a volume of between 50µl and 100µl eg 50µl or 63µl or, more preferably, 100µl.

For a 25 µg dose, when a 50µl metering volume is used, the final concentration of salmeterol delivered per actuation would be 0.05% (w/v) or 0.042% (w/w). Wherein a 63µl metering volume is used, the final concentration of salmeterol delivered per actuation would be 0.04% (w/v) or 0.033% (w/w). If a 100 µl metering valve were to be used, for a 25 µg dose the final concentration of salmeterol to delivered per actuation would be 0.025% (w/v) or 0.021% (w/w).

When a 50µl metering volume is used, the final concentration of fluticasone propionate delivered per actuation would be 0.1% w/v (which equates to 0.1g of fluticasone propionate per 100ml of formulation) or approx. 0.083% w/w (which equates to 0.083g of fluticasone propionate per 100g of formulation) for a 50µg dose, 0.25% (w/v) or approx. 0.21% (w/w) for a 125µg dose, 0.5% (w/v) or approx. 0.42% (w/w) for a 250µg dose and 0.05% (w/v) or approx 0.042% (w/w) for a 25µg dose. When a 63µl metering volume is used, the final concentration of fluticasone propionate delivered per actuation would be 0.079% (w/v) or approx. 0.067% (w/w) for a 50µg dose, 0.198% (w/v) or approx. 0.167% (w/w) for a 125µg dose, 0.397% (w/v) or approx. 0.333% (w/w) for a 250µg dose and 0.04% (w/v) or approx. 0.033% (w/w) for a 25µg dose. When a 100µl metering volume is used, the final concentration of fluticasone propionate delivered per actuation would be 0.05% w/v or approx. 0.042% w/w for a 50µg dose, 0.13% (w/v) or approx. 0.10% (w/w) for a 125µg dose, 0.25% (w/v) or approx. 0.21% (w/w) for a 250µg dose and 0.025% (w/v) or approx 0.021% (w/w) for a 25µg dose.

The previously referred to w/w figures are approximate in that they do not compensate for the density mismatch between HFA134a and ethanol, however the precise figures may be readily determined.

Use of a larger metering chamber volume eg 100µl is generally preferred.

We prefer the formulation to contain between 0.5 and 2% (w/w) of the low volatility component, more preferably between 0.8 and 1.6% (w/w), particularly between 1.0 and 1.6% (w/w). We especially prefer to use 1.3% (w/w). We also especially prefer to use 1.0% (w/w) of the low volatility component. However the most preferred range is 0.5-1% (w/w) eg 0.5%, 0.75% or 1% (w/w).

It is necessary to employ the low volatility component, the solubilising agent and the propellant in relative proportions such that the components are freely miscible.

Depending on the final concentration of salmeterol or pharmaceutically acceptable salt thereof and fluticasone propionate in the formulation, the propellant, and the precise amount of low volatility component, the concentration of solubilisation agent (eg ethanol) required will vary. So as not to suppress the vapour pressure of the propellant to an undesirable extent, the amount of ethanol should preferably not exceed around 35%. The amount of ethanol will more preferably be in the range 5 to 30%, more particularly 5-15% especially 6-12% eg 7-10% w/w.

When the concentration of salmeterol xinafoate is around 0.025% w/v, the concentration of fluticasone propionate is around 0.05% w/v and the propellant is 1,1,1,2-tetrafluoroethane, an amount of ethanol of 21-24% w/w, especially around 22% w/w is particularly suitable. When the concentration of salmeterol xinafoate is around 0.025% w/v, the concentration of fluticasone propionate is around 0.025% w/v and the propellant is 1,1,1,2-tetrafluoroethane, an amount of ethanol of 17-20% w/w, especially around 18% w/w is particularly suitable.

When the concentration of salmeterol (as xinafoate) is around 0.025% w/v, the concentration of fluticasone propionate is around 0.025% w/v and the propellant is 1,1,1,2-tetrafluoroethane, an amount of ethanol of 23-26% w/w, especially around 25% w/w is particularly suitable. When the concentration of salmeterol (as xinafoate) is around 0.025% w/v, the concentration of fluticasone propionate is around 0.05% w/v and the propellant is 1,1,1,2-tetrafluoroethane, an amount of ethanol of 21-24% w/w, especially around 23% w/w is particularly suitable. When the concentration of salmeterol (as xinafoate) is around 0.025% w/v, the concentration of fluticasone propionate is around 0.13% w/v and the propellant is 1,1,1,2-tetrafluoroethane, an amount of ethanol of 20-23% w/w, especially around 21% w/w is particularly suitable. When the concentration of salmeterol (as xinafoate) is around 0.025% w/v, the concentration of fluticasone propionate is around 0.25% w/v and the propellant is 1,1,1,2-tetrafluoroethane, an amount of ethanol of 39-42% w/w, especially around 41% w/w is particularly suitable.

The concentration of salmeterol expressed as weight of xinafoate will preferably be in the range 0.02-0.05% w/v especially 0.02-0.03% w/v eg around 0.025% w/v (equivalent to 0.014%-0.035% w/v especially 0.014%-0.021% w/v eg around 0.017% w/v expressed as weight of base). Another range of interest is 0.017-0.028% w/v eg around 0.025% w/v expressed as weight of base. The concentration of fluticasone propionate will preferably be in the range 0.02-0.2% w/v especially 0.02-0.15% w/v eg 0.025-0.13% w/v, especially 0.025-0.05% w/v.

For a salmeterol concentration of 0.025% w/v (as xinafoate) we have surprisingly found that the amount of ethanol required to solubilise salmeterol xinafoate and fluticasone propionate in a formulation in HFA134a declines as the fluticasone propionate concentration increases from 0.025% w/v to 0.13% w/v. This implies that there may be some interactior between the salmeterol (as xinafoate) and fluticasone propionate in the formulation. In light of this observation we have appreciated that the formulations employing a higher strength of fluticasone propionate may be formulated with a lower amount of ethanol than might otherwise be expected. This results in a number of advantages, for example that such formulations have a higher than anticipated fine particle mass (FPM).

Thus we prefer the ratio of the concentration of salmeterol (particularly as xinafoate) to fluticasone propionate expressed in w/v terms with weight of salmeterol being expressed as weight of free base to be in the range 1:1 to 1:6 particularly 1:2 to 1:6 eg around 1:5. In concentration terms these ratios are preferably employed when the number "1" corresponds to a concentration of around 0.025% w/v.

The above ethanol concentrations are appropriate for salmeterol xinafoate in the form of Form I polymorph. A somewhat lower concentration would be expected to be necessary for the Form II polymorph.

When the concentration of R-salmeterol (present as xinafoate) is 0.04 w/v (based on weight of R-salmeterol base), the concentration of fluticasone propionate is less than around 0.05% w/v (eg 0.025%, 0.04% or 0.05% w/v) and the propellant is 1,1,1,2-tetrafluoroethane, an amount of ethanol of 12-14% w/w eg 13% w/w is suitable. When the concentration of R-salmeterol (present as xinafoate) is 0.025 w/v (based on weight of R-salmeterol base), the concentration of fluticasone propionate is less than around 0.05% w/v (eg 0.025%, 0.04% or 0.05% w/v) and the propellant is 1,1,1,2-tetrafluoroethane, an amount of ethanol of 9-11% w/w eg 10% w/w is suitable. When the concentration of R-salmeterol (present as xinafoate) is 0.025 w/v (based on weight of R-salmeterol base), the concentration of fluticasone propionate is around 0.025% w/v and the propellant is 1,1,1,2,3,3,3-heptafluoro-n-propane, an amount of ethanol of 13-15% w/w eg 14% w/w is suitable.

When the concentration of salmeterol (present as free base) is 0.05 w/v, the concentration of fluticasone propionate is around 0.05% w/v and the propellant is 1,1,1,2-tetrafluoroethane, an amount of ethanol of around 9-11% w/w eg 10% w/w is suitable. When the concentration of salmeterol (present as free base) is 0.04 w/v, the concentration of fluticasone propionate is around 0.04% w/v and the propellant is 1,1,1,2-tetrafluoroethane, an amount of ethanol of around 8-10% w/w eg 9% w/w is suitable. When the concentration of salmeterol (present as free base) is 0.025 w/v, the concentration of fluticasone propionate is around 0.05% w/v and the propellant is 1,1,1,2-tetrafluoroethane, an amount of ethanol of around 9-11% w/w eg 10% w/w is suitable. When the concentration of salmeterol (present as free base) is 0.025 w/v, the concentration of fluticasone propionate is around 0.025% w/v and the propellant is 1,1,1,2-tetrafluoroethane, an amount of ethanol of around 6-10% preferably 6-8% w/w eg 7% w/w is suitable.

When the concentration of salmeterol (present as free base) is around 0.025% w/v, the concentration of fluticasone propionate is around 0.025% w/v and the propellant is 1,1,1,2,3,3,3-heptafluoro-n-propane, an amount of ethanol of 13-15% preferably around 14% w/w is suitable.

The preferred concentration of salmeterol (as free base) in the formulation is 0.025-0.05% w/v. The preferred concentration of fluticasone propionate in the formulation is 0.025-0.13% w/v more preferably 0.025-0.05% w/v, particularly around 0.025% w/v. When 1,1,1,2-tetrafluoroethane is the propellant the preferred concentration of ethanol as solubilising agent in the formulation is around 6-12% eg 7-10% w/w. When 1,1,1,2,3,3,3-heptafluoro-n-propane is the propellant the preferred concentration of ethanol as solubilising agent in the formulation 13-15% eg around 14% w/w.

Formulations according to the invention which are free of surfactants are preferred. Formulations according to the invention which are free of all excipients besides the solubilisation agent (ethanol), low volatility component (glycerol or polyetheyleneglycol) and the propellant are particularly preferred.

We have also surprisingly found that whereas fluticasone propionate seems quite stable to chemical degradation on storage in an ethanol/HFA134a solution, salmeterol (eg as free base or xinafoate) shows a tendency to exhibit chemical degradation. Without being limited by theory we believe that this chemical degradation may be dueto acid catalysed dimerisation of the salmeterol. Thus it may be preferred to incorporate an agent in an amount capable of suppressing chemical degradation of salmeterol in the formulation. For examples agents capable of preventing acid catalysed dimerisation include bases such as sodium or potassium hydroxide or sodium carbonate or an organic amine. It may be necessary also to incorporate a small quantity of water into the formulation eg 0.05-2% w/w water or more preferably 0.1-1% w/w water. Chemical degradation may also be promoted by oxidation eg arising from trace amounts of peroxide present in valve components (such as peroxide cured rubbers) or excipients. Preferably peroxide contamination will be avoided eg by use of appropriately cleansed valve components and the like. Alternatively an anti-oxidant may be employed (preferably one which is not an acid). Formulations according to the invention which are free of all excipients besides the solubilisation agent (ethanol), low volatility component (glycerol or polyethylene glycol, the agent capable of suppressing chemical degradation of salmeterol and any water in the formulation and the propellant are also preferred.

The pharmaceutical composition according to the present invention may be filled into canisters suitable for delivering pharmaceutical aerosol formulations. Canisters generally comprise a container capable of withstanding the vapour pressure of the HFA propellant, such as plastic or plastic-coated glass bottle or preferably a metal can, for example an aluminium can which may optionally be anodised, lacquer-coated and/or plastic-coated, which container is closed with a metering valve. Canisters may be coated with a polymer as described in WO 96/32151, for example, a co-polymer of polyethersulphone (PES) and polytetrafluoroethylene (PTFE). Another polymer for coating that may be contemplated is FEP (fluorinated ethylene propylene). The metering valves are designed to deliver a metered amount of the formulation per actuation and incorporate a gasket to prevent leakage of propellant through the valve. The gasket may comprise any suitable elastomeric material such as for example low density polyethylene, chlorobutyl, black and white butadiene-acrylonitrile rubbers, butyl rubber, neoprene, EPDM (eg as described in WO95/02651) and TPE (thermoplastic elastomer; eg as described in WO92/11190). EPDM and TPE rubbers are preferred. Suitable valves are commercially available from manufacturers well known in the aerosol industry, for example, from Valois, France (eg. DF10, DF30, DF60), Bespak plc, UK (eg. BK300, BK356, BK357) and 3M-Neotechnic Ltd, UK (eg. Spraymiser™). The DF31 valve of Valois, France is also suitable.

Valve seals, especially the gasket seal, and also the seals around the metering chamber, will preferably be manufactured of a material which is inert to and resists extraction into the contents of the formulation, especially when the contents include ethanol.

Valve materials, especially the material of manufacture of the metering chamber, will preferably be manufactured of a material which is inert to and resists distortion by contents of the formulation, especially when the contents include ethanol. Particularly suitable materials for use in manufacture of the metering chamber include polyesters eg polybutyleneterephthalate (PBT) and acetals, especially PBT.

Materials of manufacture of the metering chamber and/or the valve stem may desirably be fluorinated, partially fluorinated or impregnated with fluorine containing substances in order to resist drug deposition.

Valves which are entirely or substantially composed of metal components (eg Spraymiser, 3M-Neotechnic) are especially preferred for use according to the invention.

Conventional bulk manufacturing methods and machinery well known to those skilled in the art of pharmaceutical aerosol manufacture may be employed for the preparation of large scale batches for the commercial production of filled canisters. Thus, for example, in one bulk manufacturing method a metering valve is crimped onto an aluminium can to form an empty canister. The medicament is added to a charge vessel and a mixture of ethanol, low volatility component and liquefied propellant is pressure filled through the charge vessel into a manufacturing vessel. An aliquot of the formulation is then filled through the metering valve into the canister.

In an alternative process, an aliquot of the liquified formulation is added to an open canister under conditions which are sufficiently cold that the formulation does not vaporise. and then a metering valve crimped onto the canister.

In an alternative process an aliquot of medicament dissolved in the solubilising agent and any low-volatility component is dispensed into an empty canister, a metering valve is crimped on and then the propellant is filled into the canister through the valve.

Typically, in batches prepared for pharmaceutical use, each filled canister is check-weighed, coded with a batch number and packed into a tray for storage before release testing.

Each filled canister is conveniently fitted into a suitable channelling device prior to use to form a metered dose inhaler for administration of the medicament into the lungs or nasal cavity of a patient. Suitable channelling devices comprise, for example a valve actuator and a cylindrical or cone-like passage through which medicament may be delivered from the filled canister via the metering valve to the nose or mouth of a patient eg. a mouthpiece actuator.

In a typical arrangement the valve stem is seated in a nozzle block which has an orifice leading to an expansion chamber. The expansion chamber has an exit orifice which extends into the mouthpiece. Actuator (exit) orifice diameters in the range 0.15-0.45mm particularly 0.2-0.45mm are generally suitable eg 0.25, 0.30, 0.33 or 0.42mm. We have found that it is advantageous to use a small diameter eg 0.25mm or less, particularly 0.22mm since this tends to result in a higher FPM and lower throat deposition. 0.15mm is also particularly suitable. The dimensions of the orifice should not be so small that blockage of the jet occurs.

Actuator jet lengths are typically in the range 0.30-1.7mm eg 0.30, 0.65 or 1.50mm. Smaller dimensions are preferred eg 0.65mm or 0.30mm.

For the avoidance of water ingress into the formulation it may be desired to overwrap the MDI product in a flexible package capable of resisting water ingress and capable of permitting absorption or release of any propellant which may leak from the canister. It may also be desired to incorporate a desiccant within the packaging. Example overwraps are described in US Patent 6119853.

Metered dose inhalers are designed to deliver a fixed unit dosage of medicament per actuation or 'puff', for example in the range of 10 to 5000 µg medicament per puff.

Administration of medicament may be indicated for the treatment of mild, moderate or severe acute or chronic symptoms or for prophylactic treatment. Treatment may be of asthma, chronic obstructive pulmonary disease (COPD) or other respiratory disorder. It will be appreciated that the precise dose administered will depend upon the age and condition of the patient, the quantity and frequency of administration will ultimately be at the discretion of the attendant physician. Typically, administration may be one or more times, for example from 1 to 8 times per day, giving for example 1,2,3 or 4 puffs each time.

The preferred treatment regime is 2 puffs of 25µg/puff salmeterol (eg as xinafoate) and 25, 50, 125 or 250µg/puff (particularly 25 or 50µg/puff) fluticasone propionate, 2 times per day.

The filled canisters and metered dose inhalers described herein comprise further aspects of the present invention.

A further aspect of the present invention comprises the use of a formulation herein before described in the manufacture of a medicament for the treatment of respiratory disorders, eg. asthma or chronic obstructive pulmonary disease (COPD).

As mentioned above the advantages of the invention in some or all of its embodiments include the fact that formulations according to the invention may be more environmentally friendly, more stable, less susceptible to Ostwald ripening or drug deposition onto internal surfaces of a metered dose inhaler, have better dosing uniformity, deliver a higher FPM, give lower throat deposition, be more easily or economically manufactured, or may be otherwise beneficial relative to known formulations.

The invention is illustrated with reference to the following examples:

### Examples 1 and 2

Formulations may be prepared with composition as follows:

| | |
|---|---|
| Salmeterol xinafoate | 0.025% w/v |
| Fluticasone propionate | 0.05% w/v |
| Ethanol | 22% w/w |
| Glycerol | 1.3% w/w |
| 1,1,1,2-tetrafluoroethane | to 100% |

This solution formulation may be filled into an aluminium canister under pressure and fitted with a metering valve having a 100 µl metering chamber.

| | |
|---|---|
| Salmeterol xinafoate | 0.025% w/v |
| Fluticasone propionate | 0.025% w/v |
| Ethanol | 18% w/w |
| Glycerol | 1.3% w/w |
| 1,1,1,2-tetrafluoroethane | to 100% |

This solution formulation may be filled into an aluminium canister under pressure and fitted with a metering valve having a 100 µl metering chamber.

### Example 3

A formulation was prepared with compositions as follows:

| | |
|---|---|
| Salmeterol (as xinafoate) | 0.025% w/v (based on weight of salmeterol base) |
| Fluticasone propionate | 0.025% w/v |
| Ethanol | 25% w/w |
| Glycerol | 1.0% w/w |
| 1,1,1,2-tetrafluoroethane | to 100% |

This solution formulation was filled into an aluminium canister (120 actuations/canister; overage of 40 actuations) under pressure and fitted with a metering valve (Valois DF60) having metering chamber of volume 100 µl. This formulation is suitable for delivering 25 µg salmeterol and 25 µg fluticasone propionate per actuation.

### Example 4

A formulation was prepared with compositions as follows:

| | |
|---|---|
| Salmeterol (as xinafoate) | 0.025% w/v (based on weight of salmeterol base) |
| Fluticasone propionate | 0.05% w/v |
| Ethanol | 23% w/w |
| Glycerol | 1.0% w/w |
| 1,1,1,2-tetrafluoroethane | to 100% |

This solution formulation was filled into an aluminium canister (120 actuations/canister; overage of 40 actuations) under pressure and fitted with a metering valve (Valois DF60) having metering chamber of volume 100 µl. This formulation is suitable for delivering 25 µg salmeterol and 50 µg fluticasone propionate per actuation.

### Example 5

A formulation was prepared with compositions as follows:

| | |
|---|---|
| Salmeterol (as xinafoate) | 0.025% w/v (based on weight of salmeterol base |
| Fluticasone propionate | 0.13% w/v |
| Ethanol | 21 % w/w |
| Glycerol | 1.0% w/w |
| 1,1,1,2-tetrafluoroethane | to 100% |

This solution formulation was filled into an aluminium canister (120 actuations/canister; overage of 40 actuations) under pressure and fitted with a metering valve (Valois DF60) having metering chamber of volume 100 µl. This formulation is suitable for delivering 25 µg salmeterol and 125 µg fluticasone propionate per actuation.

### Examples 6-8

Formulations were prepared with composition as follows:

| | |
|---|---|
| Salmeterol (as free base) | 0.025% w/v |
| Fluticasone propionate | 0.025% w/v |
| Ethanol | 7% w/w |
| Glycerol or PEG200 or PEG400 | 0.5% w/w |
| 1,1,1,2-tetrafluoroethane | to 100% |

These solution formulations were filled into an aluminium canister (120 actuations/canister; overage of 40 actuations) under pressure and fitted with a metering valve (Valois DF60) having metering chamber of volume 100 µl. These formulations are suitable for delivering 25 µg salmeterol and 25 µg fluticasone propionate per actuation.

### Examples 9-11

Formulations were prepared with composition as follows:

| | |
|---|---|
| Salmeterol (as free base) | 0.025% w/v |
| Fluticasone propionate | 0.05% w/v |
| Ethanol | 10% w/w |
| Glycerol or PEG200 or PEG400 | 0.5% w/w |
| 1,1,1,2-tetrafluoroethane | to 100% |

These solution formulations were filled into an aluminium canister (120 actuations/canister; overage of 40 actuations) under pressure and fitted with a metering valve (Valois DF60) having metering chamber of volume 100 µl. These formulations are suitable for delivering 25 µg salmeterol and 50 µg fluticasone propionate per actuation.

### Andersen Cascade Impaction Data

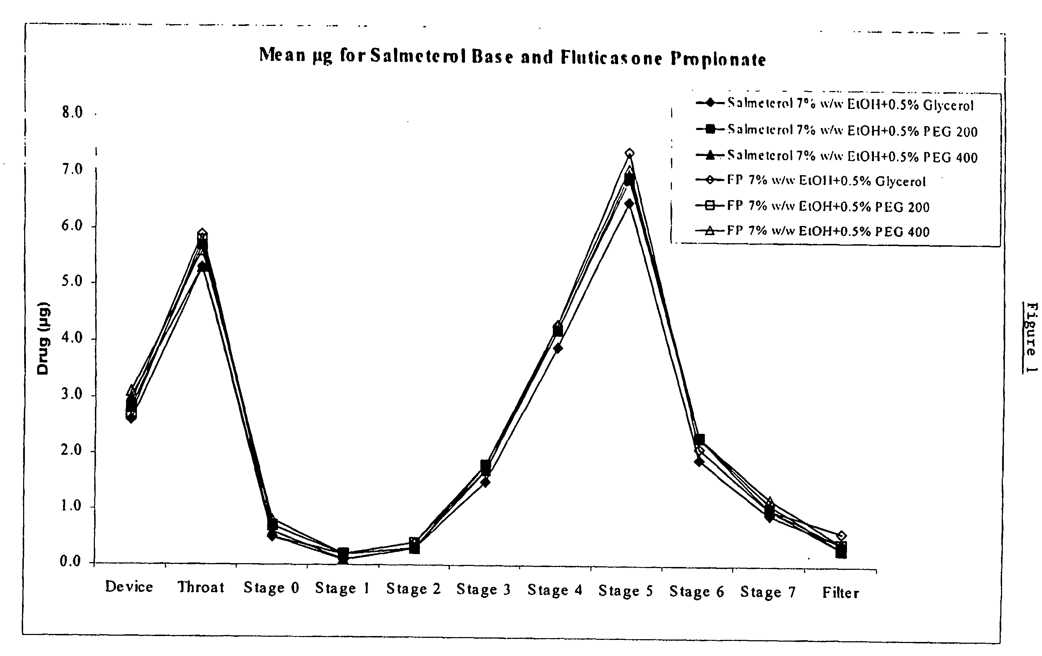

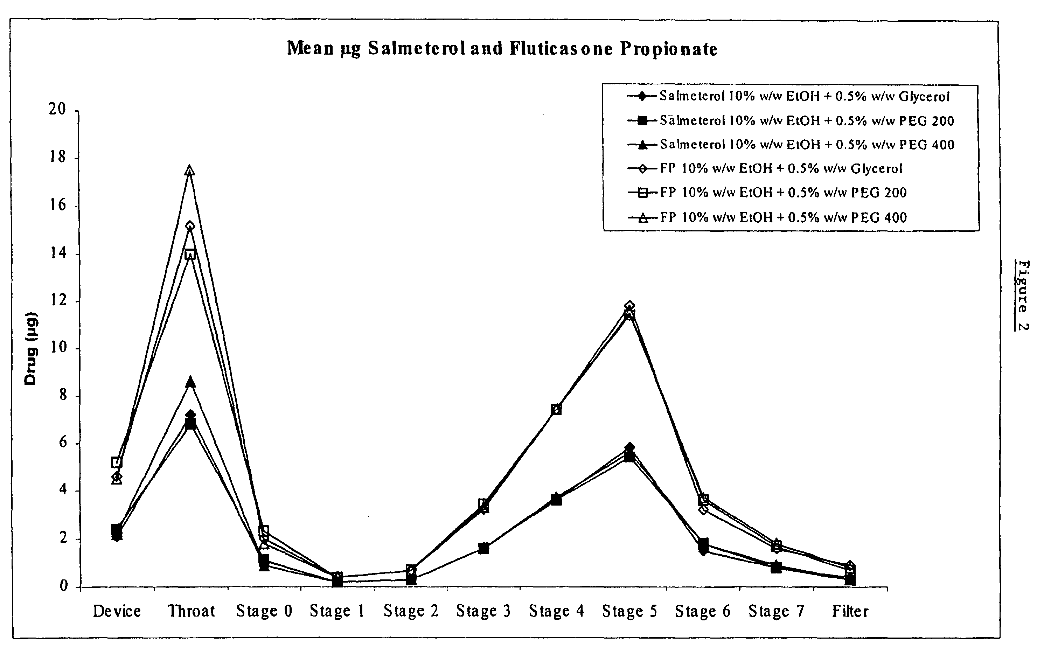

Formulations as described in Examples 6 to 11 were profiled using an Andersen Cascade Impactor, using a 0.22mm (orifice) x 0.65mm (jet length) actuator from Bespak (BK621 variant). Testing was performed on canisters at "beginning of use" (BoU) and delivered drug from 10 actuations was collected in the instrument after 4 priming actuations were fired to waste. Results are shown in Tables 1 to 4 and Figures 1 and 2. Product on the individual stages of the Cascade Impactor was dissolved up in aqueous methanol and eluted through an HPLC column (stationary phase: 20cm C₁₈ reverse phase (Hypersil); mobile phase: methanol/MeCN buffered with ammonium acetate) in order to allow the respective amounts of salmeterol and fluticasone propionate to be determined (these amounts being shown separately in the Tables and Figures below).

### Brief Description of the Tables:

Table 1: Cascade Impaction analysis of salmeterol and fluticasone propionate/HFA134a solution aerosols containing 7% ethanol with 0.5% of various low volatility components (as per Examples 6-8) (microgram data)
Table 2: Cascade Impaction analysis of salmeterol and fluticasone propionate/HFA134a solution aerosols containing 7% ethanol with 0.5% of various low volatility components (as per Examples 6-8) (percentage data)
Table 3: Cascade Impaction analysis of salmeterol and fluticasone propionate/HFA134a solution aerosols containing 10% ethanol with 0.5% of various low volatility components (as per Examples 9-11) (microgram data)
Table 4: Cascade Impaction analysis of salmeterol and fluticasone propionate/HFA134a solution aerosols containing 10% ethanol with 0.5% of various low volatility components (as per Examples 9-11) (percentage data)

### Brief Description of the Figures:

Figure 1: Cascade Impaction analysis of salmeterol and fluticasone propionate/HFA134a solution aerosols containing 7% ethanol with 0.5% of various low volatility components (microgram data) (Data as per Table 1)
Figure 2: Cascade Impaction analysis of salmeterol and fluticasone propionate/HFA134a solution aerosols containing 10% ethanol with 0.5% of various low volatility components (microgram data) (Data as per Table 3)

From the Tables and Figures it may be deduced that exceptionally good data in terms of fine particle mass is obtained from the use of salmeterol base and fluticasone propionate using ethanol as solubilising agent and HFA134a as propellant with glycerol or polyethylene glycol (PEG200, PEG400) as low volatility component. Furthermore these data on salmeterol and fluticasone propionate in solution formulation together are very similar to data on salmeterol and fluticasone propionate in solution formulation individually. They are also similar to corresponding data on the currently marketed excipient free suspension formulation of salmeterol xinafoate and fluticasone propionate in HFA134a (Seretide Evohaler).

Throughout the specification and the claims which follow, unless the context requires otherwise, the word 'comprise', and variations such as 'comprises' and 'comprising', will be understood to imply the inclusion of a stated integer or step or group of integers but not to the exclusion of any other integer or step or group of integers or steps.

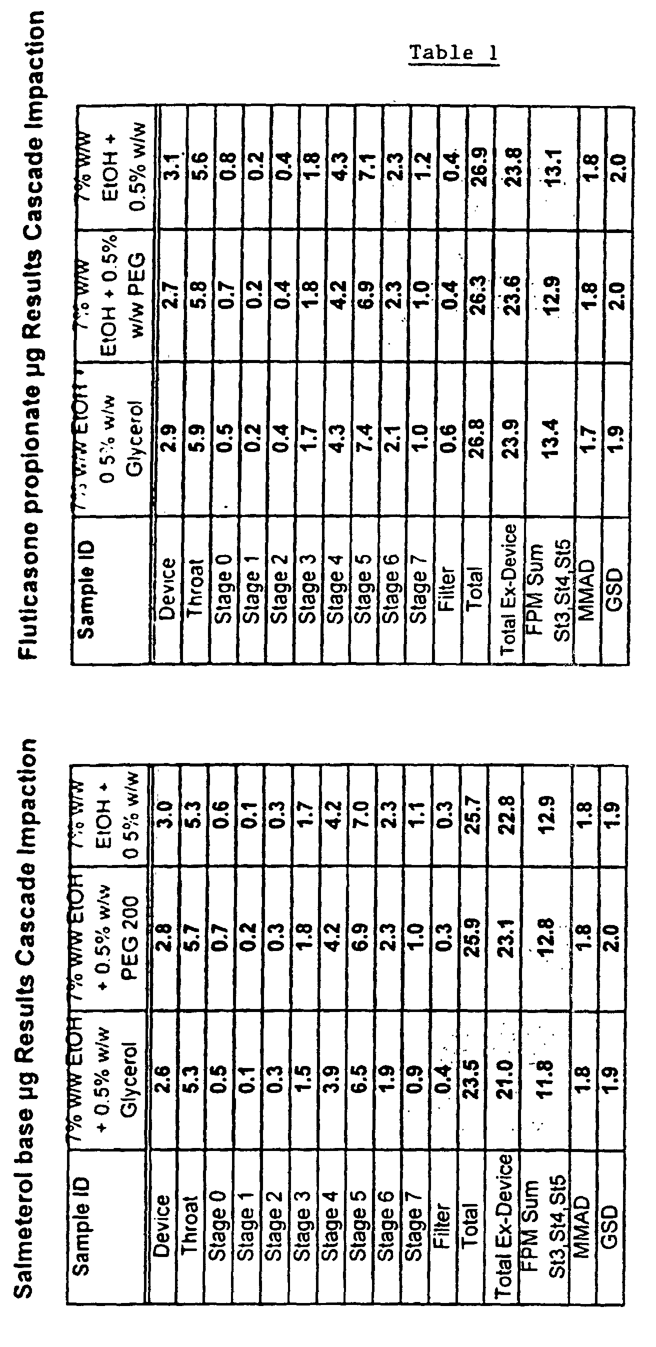

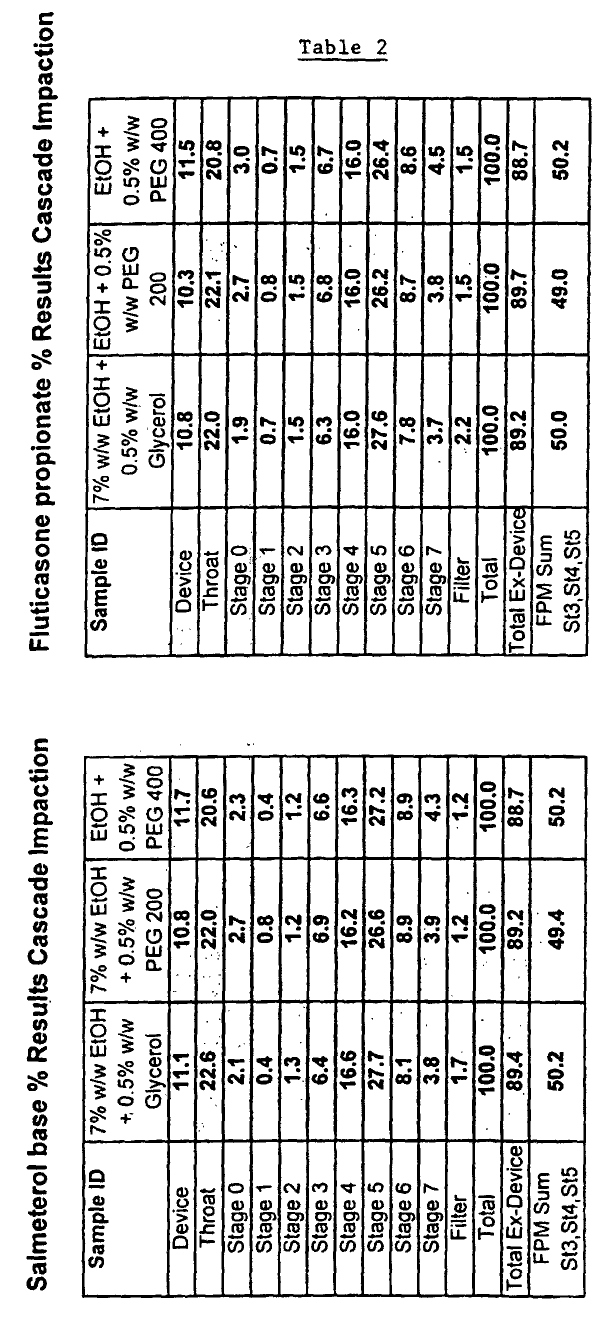

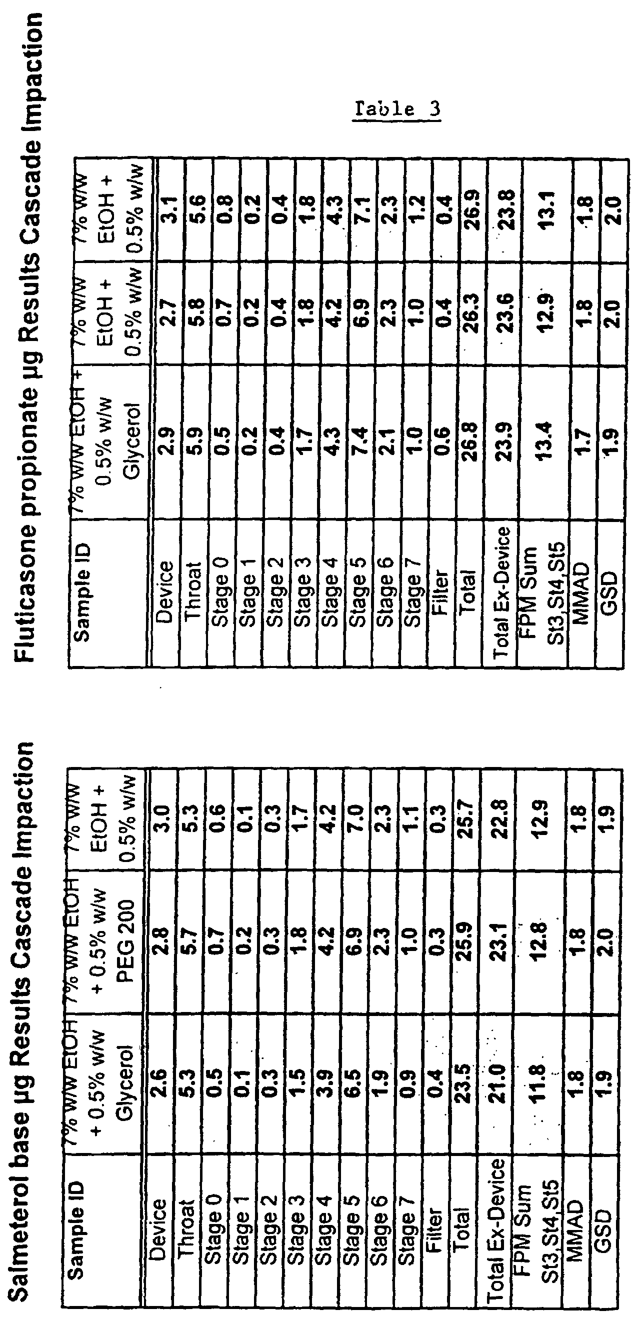

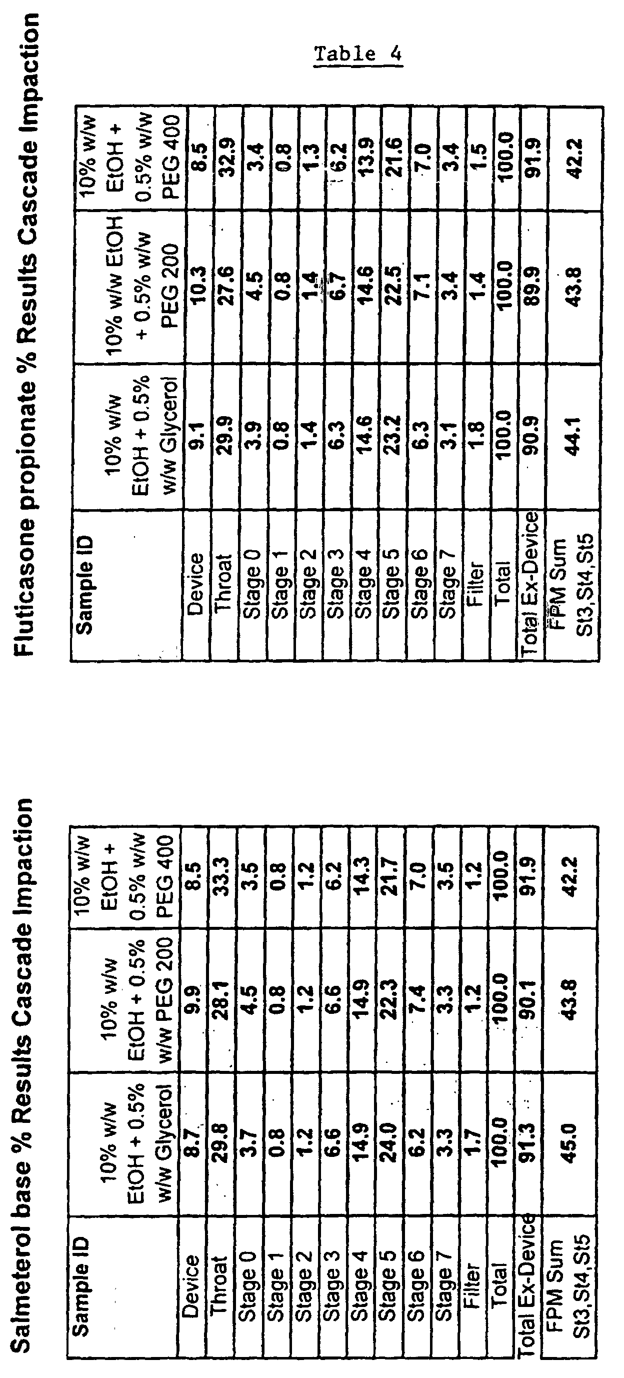

## Claims

1. A pharmaceutical aerosol formulation which comprises:
(i) salmeterol or a pharmaceutically acceptable salt thereof
(ii) fluticasone propionate
(iii) a hydrofluoroalkane (HFA) propellant
(iv) a low volatility component selected from glycerol and polyethylene glycol to increase the mass median aerodynamic diameter (MMAD) of the aerosol particles on actuation of the inhaler; and
(v) ethanol as a solubilising agent in sufficient quantity to solubilise the salmeterol or pharmaceutically acceptable salt thereof and fluticasone propionate in the formulation,
**characterised in that** the salmeterol or pharmaceutically acceptable salt thereof and fluticasone propionate are completely dissolved in the formulation.

2. A formulation according to claim 1 wherein the hydrofluoroalkane (HFA) propellant is 1,1,1,2-tetrafluoroethane (HFA134a).

3. A formulation according to claim 1 wherein the hydrofluoroalkane (HFA) propellant is 1,1,1,2,3,3,3-heptafluoro-n-propane (HFA227).

4. A formulation according to any one of claims 1 to 3 wherein the low volatility component is glycerol.

5. A formulation according to any one of claims 1 to 3 wherein the low volatility component is polyethylene glycol.

6. A formulation according to any one of claims 1 to 3 wherein the low volatility component is PEG200 or PEG400.

7. A formulation according to any one of claims 1 to 6 wherein the concentration of ethanol is 5 to 30% w/w.

8. A formulation according to any one of claims 1 to 7 wherein the concentration of ethanol is 6 to 12% w/w.

9. A formulation according to any one of claims 1 to 8 wherein salmeterol is present as salmeterol base.

10. A formulation according to any one of claims 1 to 9 wherein salmeterol is present as the xinafoate salt.

11. A formulation according to claim 10 wherein salmeterol xinafoate is present in the form of its Form II polymorph.

12. A formulation according to claims 1 to 11 wherein the concentration of salmeterol expressed as weight of xinafoate is 0.02-0.03% w/v.

13. A formulation according to claims 1 to 11 wherein the concentration of salmeterol expressed as weight of base is 0.014-0.021% w/v.

14. A formulation according to claims 1 to 11 wherein the concentration of salmeterol expressed as weight of base is 0.017-0.028% w/v.

15. A formulation according to claim 14 wherein the concentration of salmeterol expressed as weight of base is around 0.025% w/v.

16. A formulation according to claim 9 wherein the concentration of salmeterol base is 0.025-0.05% w/v.

17. A formulation according to any one of claims 1 to 16 wherein salmeterol is present as R-salmeterol.

18. A formulation according to claims 1 to 17 wherein the concentration of fluticasone propionate is in the range 0.02-0.2% w/v.

19. A formulation according to claim 18 wherein the concentration of fluticasone propionate is in the range 0.02-0.15% w/v.

20. A formulation according to any one of claims 1 to 19 wherein the ratio of the concentration of salmeterol to fluticasone propionate expressed as w/v with weight of salmeterol being expressed as weight of free base is in the range 1:1 to 1:6.

21. A formulation according to claim 20 wherein in concentration terms said ratio is employed with the number "1" corresponding to a concentration of around 0.025 w/v.

22. A formulation according to any one of claims 1 to 21 which contains a low volatility component at between 0.5 and 3% (w/w).

23. A formulation according to claim 22 which contains between 1.0 and 1.6% (w/w) of the low volatility component.

24. A formulation according to claim 22 which contains 1.0% (w/w) of the low volatility component.

25. A formulation according to claim 22 which contains between 0.5 and 1.0% (w/w) of the volatility component.

26. A formulation according to claim 1 which comprises:
(i) 0.025-0.05% w/v salmeterol base;
(ii) 0.025-0.05% w/v fluticasone propionate;
(iii) 1,1,1,2-tetrafluaroethane as propellant;
(iv) 0.5-1% w/w of a low volatility component selected from glycerol and polyethylene glycol; and
(v) 6-12% w/w ethanol as solubilising agent.

27. A formulation according to claim 26 wherein the low volatility component is PEG200 or PEG400.

28. A formulation according to claim 26 wherein the low volatility component is glycerol.

29. A formulation according to any one of claims 1 to 28 further comprising a compound capable of preventing chemical degradation of salmeterol in the formulation.

30. A canister comprising a metering valve and containing a pharmaceutical aerosol formulation according to any one of claims 1 to 29.

31. A metered dose inhaler which comprises a canister as claimed in claim 30 fitted into a suitable channelling device.

32. Use of a pharmaceutical aerosol formulation according to any one of claims 1 to 29 in the manufacture of a medicament for the treatment of respiratory disorders.

33. Use according to claim 32 wherein the respiratory disorder is asthma or chronic obstructive pulmonary disease (COPD).

## Patentansprüche

1. Pharmazeutische Aerosolformulierung, die folgendes umfasst:
(i) Salmeterol oder ein pharmazeutisch akzeptables Salz davon,
(ii) Fluticasonpropionat,
(iii) ein Hydrofluoralkan-(HFA)-Treibmittel,
(iv) eine Komponente mit geringer Flüchtigkeit, ausgewählt aus Glycerin und Polyethylenglykol, um den Massenmedianwert des aerodynamischen Durchmessers (MMAD) der Aerosolpartikel bei Auslösung des Inhalators zu erhöhen, und
(v) Ethanol als Solubilisierungsmittel in ausreichender Menge, um das Salmeterol oder pharmazeutisch akzeptable Salz davon und das Fluticasonpropionat in der Formulierung zu solubilisieren,
**dadurch gekennzeichnet, dass** das Salmeterol oder pharmazeutisch akzeptable Salz davon und das Fluticasonpropionat vollständig in der Formulierung gelöst sind.

2. Formulierung gemäss Anspruch 1, worin das Hydrofluoralkan-(HFA)-Treibmittel 1,1,1,2-Tetrafluorethan (HFA134a) ist.

3. Formulierung gemäss Anspruch 1, worin das Hydrofluoralkan-(HFA)-Treibmittel 1,1,1,2,3,3,3-Heptafluor-n-propan (HFA227) ist.

4. Formulierung gemäss einem der Ansprüche 1 bis 3, worin die Komponente mit geringer Flüchtigkeit Glycerin ist.

5. Formulierung gemäss einem der Ansprüche 1 bis 3, worin die Komponente mit geringer Flüchtigkeit Polyethylenglykol ist.

6. Formulierung gemäss einem der Ansprüche 1 bis 3, worin die Komponente mit geringer Flüchtigkeit PEG200 oder PEG400 ist.

7. Formulierung gemäss einem der Ansprüche 1 bis 6, worin die Ethanolkonzentration 5 bis 30 % G/G ist.

8. Formulierung gemäss einem der Ansprüche 1 bis 7, worin die Ethanolkonzentration 6 bis 12 % G/G ist.

9. Formulierung gemäss einem der Ansprüche 1 bis 8, worin Salmeterol als Salmeterolbase vorhanden ist.

10. Formulierung gemäss einem der Ansprüche 1 bis 9, worin Salmeterol als Xinafoatsalz vorhanden ist.

11. Formulierung gemäss Anspruch 10, worin Salmeterolxinafoat in Form seines Polymorphs der Form II vorhanden ist.

12. Formulierung gemäss Ansprüchen 1 bis 11, worin die Salmeterolkonzentration, ausgedrückt als Masse von Xinafoat, 0,02 bis 0,03 % G/V ist.

13. Formulierung gemäss Ansprüchen 1 bis 12, worin die Salmeterolkonzentration, ausgedrückt als Masse der Base, 0,014 bis 0,021 % G/V ist.

14. Formulierung gemäss Ansprüchen 1 bis 11, worin die Salmeterolkonzentration, ausgedrückt als Masse der Base, 0,017 bis 0,028 % G/V ist.

15. Formulierung gemäss Anspruch 14, worin die Salmeterolkonzentration, ausgedrückt als Masse der Base, ca. 0,025 % G/V ist.

16. Formulierung gemäss Anspruch 9, worin die Konzentration an Salmeterolbase 0,025 bis 0,05 % G/V ist.

17. Formulierung gemäss einem der Ansprüche 1 bis 16, worin das Salmeterol als R-Salmeterol vorhanden ist.

18. Formulierung gemäss Ansprüchen 1 bis 17, worin die Konzentration an Fluticasonpropionat im Bereich von 0,02 bis 0,2 % G/V ist.

19. Formulierung gemäss Anspruch 18, worin die Konzentration an Fluticasonpropionat im Bereich von 0,02 bis 0,15 % G/V ist.

20. Formulierung gemäss einem der Ansprüche 1 bis 19, worin das Konzentrationsverhältnis von Salmeterol zu Fluticasonpropionat, ausgedrückt als G/V, wobei die Masse von Salmeterol als Masse der freien Base ausgedrückt wird, im Bereich von 1:1 bis 1:6 ist.

21. Formulierung gemäss Anspruch 20, worin das Verhältnis in bezug auf die Konzentration mit der Zahl "1" eingesetzt wird, die einer Konzentration von ca. 0, 025 G/V entspricht.

22. Formulierung gemäss einem der Ansprüche 1 bis 21, die eine Komponente mit geringer Flüchtigkeit mit zwischen 0,5 und 3 % (G/G) enthält.

23. Formulierung gemäss Anspruch 22, die zwischen 1,0 und 1,6 % (G/G) der Komponente mit geringer Flüchtigkeit enthält.

24. Formulierung gemäss Anspruch 22, die 1,0 % (G/G) der Komponente mit geringer Flüchtigkeit enthält.

25. Formulierung gemäss Anspruch 22, die zwischen 0,5 und 1,0 % (G/G) der Komponente mit geringer Flüchtigkeit enthält.

26. Formulierung gemäss Anspruch 1, die folgendes umfasst:
(i) 0,025 bis 0,05 % G/V Salmeterolbase;
(ii) 0,025 bis 0,05 % G/V Fluticasonpropionat;
(iii) 1,1,1,2-Tetrafluorethan als Treibmittel;
(iv) 0,5 bis 1 % G/G einer Komponente mit geringer Flüchtigkeit, ausgewählt aus Glycerin und Polyethylenglykol; und
(v) 6 bis 12 % G/G Ethanol als Solubilisierungsmittel.

27. Formulierung gemäss Anspruch 26, worin die Komponente mit geringer Flüchtigkeit PEG200 oder PEG400 ist.

28. Formulierung gemäss Anspruch 26, worin die Komponente mit geringer Flüchtigkeit Glycerin ist.

29. Formulierung gemäss einem der Ansprüche 1 bis 28, die ferner eine Verbindung umfasst, die den chemischen Abbau von Salmeterol in der Formulierung verhindern kann.

30. Dose, die ein Dosierventil umfasst und eine pharmazeutische Aerosolformulierung gemäss einem der Ansprüche 1 bis 29 enthält.

31. Dosierinhalator, der eine Dose gemäss Anspruch 30 umfasst, eingepasst in eine geeignete Kanalisierungsvorrichtung.

32. Verwendung einer pharmazeutischen Aerosolformulierung gemäss einem der Ansprüche 1 bis 29 in der Herstellung eines Medikaments zur Behandlung von Atemwegsstörungen.

33. Verwendung gemäss Anspruch 32, worin die Atemwegsstörung Asthma oder chronisch-obstruktive Lungenkrankheit (COPD) ist.

## Revendications

1. Formulation pharmaceutique en aérosol qui comprend :
(i) du salmétérol ou un de ses sels pharmaceutiquement acceptables
(ii) du propionate de fluticasone
(iii) un gaz propulseur hydrofluoroalcane (HFA)
(iv) un composant de faible volatilité sélectionné parmi le glycérol et le polyéthylèneglycol pour augmenter le diamètre aérodynamique massique médian (DAMM) des particules aérosol après actionnement de l'inhalateur ; et
(v) de l'éthanol en tant qu'agent de solubilisation en quantité suffisante pour solubiliser le salmétérol ou un de ses sels pharmaceutiquement acceptables et le propionate de fluticasone dans la formulation,
**caractérisée en ce que** le salmétérol ou un de ses sels pharmaceutiquement acceptable et le propionate de fluticasone sont complètement dissous dans la formulation.

2. Formulation selon la revendication 1, dans laquelle le gaz propulseur hydrofluoroalcane (HFA) est le 1,1,1,2-tétrafluoroéthane (HFA134a).

3. Formulation selon la revendication 1, dans laquelle le gaz propulseur hydrofluoroalcane (HFA) est le 1,1,1,2,3,3,3-heptafluoro-n-propane (HFA227).

4. Formulation selon l'une quelconque des revendications 1 à 3 dans laquelle le composant de faible volatilité est le glycérol.

5. Formulation selon l'une quelconque des revendications 1 à 3 dans laquelle le composant de faible volatilité est le polyéthylèneglycol.

6. Formulation selon l'une quelconque des revendications 1 à 3 dans laquelle le composant de faible volatilité est PEG200 ou PEG400.

7. Formulation selon l'une quelconque des revendications 1 à 6 dans laquelle la concentration de l'éthanol est de 5 à 30 % p/p.

8. Formulation selon l'une quelconque des revendications 1 à 7 dans laquelle la concentration de l'éthanol est de 6 à 12 % p/p.

9. Formulation selon l'une quelconque des revendications 1 à 8 dans laquelle le salmétérol est présent en tant que base salmétérol.

10. Formulation selon l'une quelconque des revendications 1 à 9 dans laquelle le salmétérol est présent en tant que sel de xinafoate.

11. Formulation selon la revendication 10 dans laquelle le xinafoate de salmétérol est présent sous sa forme polymorphe II.

12. Formulation selon les revendications 1 à 11 dans laquelle la concentration de salmétérol exprimée en tant que poids du xinafoate est de 0,02 à 0,03 % p/v.

13. Formulation selon les revendications 1 à 11 dans laquelle la concentration de salmétérol exprimée en tant que poids de la base est de 0,014 à 0,021 % p/v.

14. Formulation selon les revendications 1 à 11 dans laquelle la concentration de salmétérol exprimée en tant que poids de la base est de 0,017 à 0,028 % p/v.

15. Formulation selon la revendication 14 dans laquelle la concentration de salmétérol exprimée en tant que poids de la base est d'environ 0,025 % p/v.

16. Formulation selon la revendication 9 dans laquelle la concentration de base de salmétérol est de 0,025 à 0,05 % p/v.

17. Formulation selon l'une quelconque des revendications 1 à 16 dans laquelle le salmétérol est présent en tant que R-salmétérol.

18. Formulation selon les revendications 1 à 17 dans laquelle la concentration de propionate de fluticasone est dans la gamme de 0,02 à 0,2 % p/v.

19. Formulation selon la revendication 18 dans laquelle la concentration de propionate de fluticasone est dans la gamme de 0,02 à 0,15 % p/v.

20. Formulation selon l'une quelconque des revendications 1 à 19 dans laquelle le rapport de la concentration de salmétérol au propionate de fluticasone exprimé en p/v, le poids de salmétérol étant exprimé en tant que poids de base libre est dans la gamme de 1:1 à 1:6.

21. Formulation selon la revendication 20 dans laquelle en terme de concentration, ledit rapport est employé avec le chiffre "1" correspondant à une concentration d'environ 0,025 p/v.

22. Formulation selon l'une quelconque des revendications 1 à 21 qui contient un composant de faible volatilité à entre 0,5 et 3 % (p/p).

23. Formulation selon la revendication 22 qui contient entre 1,0 et 1,6 % (p/p) du composant de faible volatilité.

24. Formulation selon la revendication 22 qui contient 1,0 % (p/p) du composant de faible volatilité.

25. Formulation selon la revendication 22 qui contient entre 0,5 et 1,0 % (p/p) du composant de faible volatilité.

26. Formulation selon la revendication 1 qui comprend :
(i) 0,025 à 0,05 % p/v de base salmétérol ;
(ii) 0,025 à 0,05 % p/v de propionate de fluticasone
(iii) du 1,1,1,2-tétrafluoroéthane en tant que gaz propulseur ;
(iv) 0,5 à 1 % p/p d'un composant de faible volatilité sélectionné parmi le glycérol et le polyéthylèneglycol ; et
(v) 6 à 12 % p/p d'éthanol en tant qu'agent de solubilisation.
(vi)

27. Formulation selon la revendication 26 dans laquelle le composant de faible volatilité est PEG200 ou PEG400.

28. Formulation selon la revendication 26 dans laquelle le composant de faible volatilité est le glycérol.

29. Formulation selon l'une quelconque des revendications 1 à 28 comprenant de plus un composé capable d'empêcher la dégradation chimique du salmétérol dans la formulation.

30. Boîte métallique comprenant une valve doseuse et contenant une formulation pharmaceutique en aérosol selon l'une quelconque des revendications 1 à 29.

31. Inhalateur doseur qui comprend une boîte métallique tel que revendiqué dans la revendication 30 placé dans un dispositif de répartition convenable.

32. Utilisation d'une formulation pharmaceutique en aérosol selon l'une quelconque des revendications 1 à 29 dans la fabrication d'un médicament pour le traitement des troubles respiratoires.

33. Utilisation selon la revendication 32, dans laquelle le trouble respiratoire est l'asthme ou une bronchopneumopathie chronique obstructive (COPD).
